# EUROPÄISCHE PATENTSCHRIFT

(11) **EP 1 330 230 B1**
(45) Veröffentlichungstag und Bekanntmachung des Hinweises auf die Patenterteilung: **29.11.2006**
(21) Anmeldenummer: 01988577.1
(22) Anmeldetag: 18.10.2001
(51) Int. Cl.: A61K 8/34, A61Q 19/00

(54) **VERWENDUNG VON FETTALKOHOLEN ALS SOLUBILISIERUNGSMITTEL**
USE OF FATTY ALCOHOLS AS SOLUBILIZING AGENTS
UTILISATION D'ALCOOLS GRAS COMME AGENTS DE SOLUBILISATION

(30) Priorität: 27.10.2000 DE 10053328
(43) Veröffentlichungstag der Anmeldung: 30.07.2003
(73) Patentinhaber: Cognis IP Management GmbH, 40589 Düsseldorf (DE)
(72) Erfinder: SEIPEL, Werner, 40723 Hilden (DE); BOYXEN, Norbert, 47906 Kempen (DE)
(86) Internationale Anmeldenummer: PCT/EP2001/012072
(87) Internationale Veröffentlichungsnummer: WO 2002/034216

(56) Entgegenhaltungen:
- EP-A- 0 211 555
- EP-A- 0 471 606
- WO-A-94/23696
- WO-A-98/04240
- WO-A-98/24402
- "Produktübersicht von A bis Z" 1998 , HENKEL KGAA , DÜSSELDORF XP002189447 Seite 19 Seite 21 Seite 31 Seite 40 Seite 41 Seite 45

## Beschreibung

### Gebiet der Erfindung

Die Erfindung befindet sich auf dem Gebiet der Kosmetik und betrifft neue kosmetische Zubereitungen in Form konzentrierter Abmischungen, bestehend aus Fettalkoholen und Wirkstoffen sowie die Verwendung von Fettalkoholen als Solubilisierungsmittel.

### Stand der Technik

Im Bereich der Körperreinigung und Körperpflege werden tensidhaltige Formulierungen eingesetzt, wobei den grenzflächenaktiven Stoffen die Aufgabe zukommt, Verunreinigungen von Haut und Haaren zu entfernen und zu solubilisieren, so dass es nicht zu einer Wiederablagerung kommt. Allerdings ist der Einsatz von Tensiden mit dem Nachteil verbunden, dass zusammen mit der Reinigung auch eine Entfettung stattfindet, die beispielsweise zu Hautrauhigkeit führt und somit vom Verbraucher als unangenehm empfunden wird. Zu diesem Zweck werden entsprechenden Präparaten als Wirkstoffe sogenannte Rückfetter zugesetzt, bei denen es sich im einfachsten Fall um Fette, Öle oder Wachse handelt, die den Lipidgehalt der Haut wieder ausgleichen.

Die Einarbeitung solcher Stoffe in wäßrige Systeme ist wegen ihrer hohen Hydrophobie alles andere als einfach. Eine Möglichkeit besteht darin, die Wirkstoffe aufzuschmelzen und dann feinstverteilt einzuarbeiten, was aber auch schon bei geringen Einsatzmengen zu einem sehr aufwendigen Verarbeitungsprozeß führt. In der Formulierungstechnik geht man daher in der Regel einen anderen Weg und solubilisiert die rückfettenden Wirkstoffe durch Einsatz von Lösungsvermittlern oder Hydrotropen. Hierbei handelt es sich jedoch üblicherweise um ethylenoxidhaltige Produkte, die eine geringe Neigung zum biologischen Abbau aufweisen, Viskosität und Schaumbildung nachteilig beeinflussen und außerdem zu einem trockenen Hautgefühl beitragen, da sie das hauteigene Sebum im Waschprozess mitlösen und so der Rückfettung gerade entgegen wirken.
Die Aufgabe der vorliegenden Erfindung hat somit darin bestanden, einen Weg aufzuzeigen, auf dem man hinreichende Mengen von Wirkstoffen, vorzugsweise rückfettende Wirkstoffe mit fehlender oder sehr geringer Wasserlöslichkeit, in wäßrige Tensidsysteme einarbeiten kann und dabei die eingangs geschilderten Nachteile des Stands der Technik zuverlässig vermeidet. Insbesondere sollte ein Lösungsmittel für die genannten Wirkstoffe gefunden werden, welches selbst rückfettende Eigenschaften besitzt, die Viskosität und Schaumbildung nicht nachteilig beeinflußt und eine ausreichende biologische Abbaubarkeit besitzt.

### Beschreibung der Erfindung

Gegenstand der Erfindung sind neue kosmetische Zubereitungen, bestehend aus
(a) 40 bis 99 Gew.-% Fettalkoholen der Formel **(I)**

   **R**^{**1**}**OH** **(I)**

   in der R¹ für einen aliphatischen, linearen oder verzweigten Kohlenwasserstoffrest mit 6 bis 22 Kohlenstoffatomen und 0 und/oder 1, 2 oder 3 Doppelbindungen steht und
(b) 1 bis 60 Gew.-% Wirkstoffen mit einer Wasserlöslichkeit bei 20 °C von weniger als 1 g/l, die ausgewählt sind aus der Gruppe, die gebildet wird von Fettsäuren, Ölkörpern, Fetten und Wachsen, Partialglyceriden sowie biogenen aktiven Systemen,
mit der Maßgabe, dass sich die Mengenangaben zu 100 Gew.-% ergänzen.

Überraschenderweise wurde gefunden, dass sich strukturell ganz unterschiedliche Wirkstoffe, denen jedoch gemeinsam ist, dass sie wasserunlöslich bzw. praktisch wasserunlöslich sind, klar, flüssig und im Rahmen einer Kaltvermischung in Fettalkohole einarbeiten lassen und diese Mischungen ("Compounds") ihrerseits wieder zusammen mit Tensiden kalt formuliert werden können, wobei die Endprodukte, beispielsweise Shampoos, Duschbäder, Schaumbäder und dergleichen, ihrerseits wieder klar sind und auch bei längerer Temperaturbelastung hinreichend stabil sind. Die Compounds, die selbst auch als Rückfettungsmittel bezeichnet werden können, zeichnen sich durch die Mitverwendung von Fettalkoholen anstelle von nichtionischen, ethylenoxidhaltigen Solubilisatoren zusätzlich durch eine ausgezeichnete biologische Abbaubarkeit sowie verbesserte rückfettende Eigenschaften aus. In den Endformulierungen beeinflussen sie weder die Viskosität noch die Schaumbildung nachteilig.

### Fettalkohole

Unter Fettalkoholen sind primäre aliphatische Alkohole der Formel **(I)** zu verstehen,

**R**^{**1**}**OH** **(I)**

in der R¹ für einen aliphatischen, linearen oder verzweigten Kohlenwasserstoffrest mit 6 bis 22, vorzugsweise 8 bis 18 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Im Hinblick auf eine besonders vorteilhafte rückfettende Wirkung hat sich der Einsatz von Fettalkoholen mit 12, 14 oder 16 Kohlenstoffatomen oder die Verwendung entsprechender Gemische, beispielsweise technische Kokosfettalkoholschnitte, als vorteilhaft erwiesen.

### Fettsäuren

Unter Fettsäuren, die als Wirkstoffe die Komponente (b1) darstellen, sind aliphatische Carbonsäuren der Formel **(II)** zu verstehen,

**R**^{**2**}**CO-OH** **(II)**

in der R²CO für einen aliphatischen, linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, I-sotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen, wie beispielsweise Kokos-, Palm-, Palmkern- oder Talgfettsäure.

### Ölkörper

Als Ölkörper (Komponente b2) kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Sheabutter, Ester von C₆-C₂₂-Fettalkoholen und/oder Guerbetalkoholen mit aromatischen Carbonsäuren, insbesondere Benzoesäure, Ester von C₂-C₁₂₋Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆₋c₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C- Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. Squalan, Squalen oder Dialkylcyclohexane in Betracht.

### Fette und Wachse

Als Komponente (b3) kommen Fette und Wachse in Betracht. Typische Beispiele für Fette sind Glyceride, d.h. feste oder flüssige pflanzliche oder tierische Produkte, die im wesentlichen aus gemischten Glycerinestern höherer Fettsäuren mit 12 bis 22 Kohlenstoffatomen bestehen. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

### Partialglyceride

Als weitere Komponente (b5) kommen Partialglyceride, also Monoglyceride, Diglyceride und deren technische Gemische in Frage, die herstellungsbedingt noch geringe Mengen Triglyceride enthalten können. Die Partialglyceride folgen vorzugsweise der Formel **(III),** 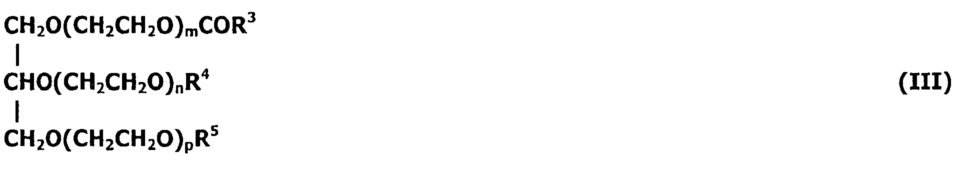 in der R³CO für einen linearen oder verzweigten, gesättigten und/oder ungesättigten Acylrest mit 6 bis 22, vorzugsweise 12 bis 18 Kohlenstoffatomen, R⁴ und R⁵ unabhängig voneinander für R⁴CO oder OH und die Summe (m+n+p) für 0 oder Zahlen von 1 bis 100, vorzugsweise 5 bis 25 steht, mit der Maßgabe, dass mindestens einer der beiden Reste R⁴ und R⁵ OH bedeutet. Typische Beispiele sind Mono- und/oder Diglyceride auf Basis von Capronsäure, Caprylsäure, 2-Ethylhexansäure, Caprinsäure, Laurinsäure, I-sotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen.

### Biogene aktive Systeme

Schließlich können als Komponente (b6) auch eine Reihe von wasserunlöslichen Biowirkstoffen eingesetzt werden, die ebenfalls über pflegende und/oder rückfettende Eigenschaften verfügen. Hierzu zählen vor allem Tocopherole, Tocopherolester, Sterole, Sterolester, Bisabolol sowohl Ceramide und Stoffe mit ceramidähnlicher Struktur, die im allgemeinen als Pseudoceramide bezeichnet werden.

Das Einsatzverhältnis der Komponenten (a) und (b) beträgt üblicherweise 40 : 60 bis 99 : 1, vorzugsweise 50 : 50 bis 90 : 10 und insbesondere 60 : 40 bis 80 : 20. Dabei versteht es sich, dass die Komponente (a) C-kettenrein oder eine Mischung verschiedener Fettalkohole darstellen kann, und mit der Komponente (b) sowohl einzelne Vertreter der Untergruppen (b1) bis (b6), als auch beliebige Mischungen gemeint sind. Die Einsatzmenge der erfindungsgemäßen Mittel bezogen auf die vorzugsweise tensidischen Endformulierungen kann 0,1 bis 10, vorzugsweise 0,5 bis 5 und insbesondere 1 bis 3 Gew.-% betragen.

### Kosmetische Zubereitungen

Ein weiterer Gegenstand der vorliegenden Erfindung betrifft die Verwendung von Fettalkoholen als Solubilisierungsmittel für Wirkstoffe mit einer Wasserlöslichkeit bei 20 °C von weniger als 1 g/l zur Herstellung von kosmetischen Zubereitungen.

Unter kosmetischen Zubereitungen sind vorzugsweise, aber nicht ausschließlich, tensidische Endformulierungen, wie z.B. Haarshampoos, Haarlotionen, Schaumbäder, Duschbäder und dergleichen zu verstehen. Es kann sich aber auch um Cremes, Gele, Lotionen, alkoholische und wäßrig/alkoholische Lösungen, Emulsionen, Wachs/ Fett-Massen oder Stiftpräparaten handeln. Diese Mittel können neben den bereits aufgeführten Komponenten (b1) bis (b6) als weitere Hilfs- und Zusatzstoffe milde Tenside, Emulgatoren, Perlglanzwachse, Konsistenzgeber, Verdickungsmittel, Überfettungsmittel, Stabilisatoren, Polymere, UV-Licht-schutzfaktoren, Antioxidantien, Deodorantien, Antitranspirantien, Antischuppenmittel, Filmbildner, Quellmittel, pflanzliche und marine Wirkstoffe, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, AIkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie Addukte mit 1 bis 30 Mol Ethylenoxid an Partialester von Glycerin und/oder Sorbitan;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
> Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

### ➢ Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

### ➢ Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### ➢ Sorbitanester

Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische in Frage. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### ➢ Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoff-atomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie weitere Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht, die jedoch schon im wesentlichen Bestandteile der erfindungsgemäßen Compounds sind. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyl-oligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B.

Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octyl-acrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der **EP 0693471 B1** beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der **EP 0694521 B1** beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strählung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Pflanzliche und marine Wirkstoffe

Unter pflanzlichen und marinen Wirkstoffen sind beispielsweise (Desoxy)Ribonucleinsäure und deren Fragmentierungsprodukte, β-Glucane, Retinol, Allantoin, Phytantriol, Panthenol, AHA-Säuren, Aminosäuren, essentielle Öle, Pflanzenextrakte, wie z.B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2' -hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremono-ethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### ➢ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkoniumtetrachlorohydrat, Aluminium-Zirkoniumpentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Verschiedene Zubereitungen auf Basis von Fettalkohol bzw. einem nichtionischen Solubilisator des Stands der Technik wurden durch Kaltverrühren der flüssigen bzw. aufgeschmolzenen Inhaltsstoffe (20 °C) hergestellt und für 4 Wochen bei 40°C gelagert. Anschließend wurde das optische Erscheinungsbild [(+) = klar; (o) = trüb] sowie die Stabilität [(+) = stabil; (o) = getrennt] beurteilt. Die gleichen Zubereitungen wurden in einer Menge von 2 Gew.-% bei 20°C einer 30 gew.-%igen wäßrigen Lösung aus Sodium Laureth Sulfate und Cocamidopropyl Betaine (1:1) zugesetzt. Wiederum wurde die Klarlöslichkeit sowie der Einfluss auf die Viskosität und das Schaumvermögen [(+) = keine Änderung von Viskosität -bzw. Schaumhöhe, (-) = Viskositätsänderung bzw. geringeres Schaumvermögen] beurteilt. Die Ergebnisse sind in Tabelle 1 zusammengefaßt. Die Beispiele 1 bis 4 sind erfindungsgemäß, die Beispiele V1 bis V4 dienen zum Vergleich.

**Tabelle 1**

| **Rückfettungscompounds** | | | | | | | | |
|---|---|---|---|---|---|---|---|---|
| **Zusammensetzung** | **1** | **2** | **3** | **4** | **V1** | **V2** | **V3** | **V4** |
| Lauryl/Myristyl Alcohol | 80 | 95 | 98 | 45 | - | - | - | - |
| PEG40 Hydrogenated Castor Oil | - | - | - | - | 80 | 95 | 98 | 45 |
| Lauric/Myristic Acid | - | - | - | 45 | - | - | - | 45 |
| Glycerol Oleate | 20 | - | - | 7 | 20 | - | - | 7 |
| Squalan | - | 5 | - | 2 | - | 5 | - | 2 |
| PEG2 Phytosterol | - | - | - | 1 | - | - | - | 1 |
| Tocopherol | - | - | 2 | - | - | - | 2 | - |
| ***Löslichkeit*** | + | + | + | + | + | + | + | ○ |
| ***Löslichkeit in Tensiden*** | + | + | + | + | ○ | ○ | + | ○ |
| ***Stabilität*** | + | + | + | + | ○ | ○ | ○ | ○ |
| ***Viskosität*** | + | + | + | + | ○ | ○ | ○ | ○ |
| ***Schaumvermögen*** | + | + | + | + | ○ | ○ | ○ | ○ |

In der folgenden Tabelle 2 sind eine Reihe von Anwendungsbeispielen zusammengefaßt.

**Tabelle 2**

| **Kosmetische Zubereitungen (Wasser, Konservierungsmittel ad 100 Gew.-%)** | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon® NSO** | - | - | - | - | - | - | 38,0 | 38,0 | 25,0 | - |
| Sodium Laureth Sulfate | | | | | | | | | | |
| **Texapon® SB 3** | - | - | - | - | - | - | - | - | 10,0 | - |
| Disodium Laureth Sulfosuccinate | | | | | | | | | | |
| **Plantacare® 818** | - | - | - | - | - | - | 7,0 | 7,0 | 6,0 | - |
| Coco Glucosides | | | | | | | | | | |
| **Plantacare® PS 10** | - | - | - | - | - | - | - | - | - | 16,0 |
| Sodium Laureth Sulfate (and) Coco Glucosides | | | | | | | | | | |
| **Dehyton® PK 45** | - | - | - | - | - | - | - | - | 10,0 | - |
| Cocamidopropyl Betaine | | | | | | | | | | |
| **Dehyquart® A** | 2,0 | 2,0 | 2,0 | 2,0 | 4,0 | 4,0 | - | - | - | - |
| Cetrimonium Chloride | | | | | | | | | | |
| **Dehyquart L® 80** | 1,2 | 1,2 | 1,2 | 1,2 | 0,6 | 0,6 | - | - | - | - |
| Dicocoylmethylethoxymonium Methosulfate (and) Propylenglycol | | | | | | | | | | |
| **Eumulgin® B2** | 0,8 | 0,8 | - | 0,8 | - | 1,0 | - | - | - | - |
| Ceteareth-20 | | | | | | | | | | |
| **Eumulgin® VL 75** | - | - | 0,8 | - | 0.8 | - | - | - | - | - |
| Lauryl Glucoside (and) Polyglyceryl-2 Polyhydroxystearate (and) Glycerin | | | | | | | | | | |
| **Lanette® O** | 2,5 | 2,5 | 2,5 | 2,5 | 3,0 | 2,5 | - | - | - | - |
| Cetearyl Alcohol | | | | | | | | | | |
| **Cutina® GMS** | 0,5 | 0,5 | 0,5 | 0,5 | 0,5 | 1,0 | - | - | - | - |
| Glyceryl Stearate | | | | | | | | | | |
| **Cetiol® HE** | 1,0 | - | - | - | - | - | - | - | 1,0 | |
| PEG-7 Glyceryl Cocoate | | | | | | | | | | |
| **Cetiol® PGL** | - | 1,0 | - | - | 1,0 | - | - | - | - | - |
| Hexyldecanol (and) Hexyldecyl Laurate | | | | | | | | | | |
| **Cetiol® V** | - | - | - | 1,0 | - | - | - | - | - | - |
| Decyl Oleate | | | | | | | | | | |
| **Eutanol® G** | - | - | 1,0 | - | - | 1,0 | - | - | - | - |
| Octyldodecanol | | | | | | | | | | |
| **Nutrilan® Keratin W** | - | - | - | 2,0 | - | - | - | - | - | - |
| Hydrolyzed Keratin | | | | | | | | | | |
| **Euperlan® PK 3000 AM** | - | - | - | - | - | - | - | 3,0 | 5,0 | 5,0 |
| Glycol Distearate (and) Laureth-4 (and) Cocamidopropyl Betaine | | | | | | | | | | |
| **Rückfettungscompound gem. Bsp. 4** | 2,0 | 2,0 | 2,0 | 2,0 | 2,0 | 2,0 | 2,0 | 2,0 | 2,0 | 2,0 |
| **Hydagen® CMF** | 1,0 | 1,0 | 1,0 | 1,0 | 1,0 | 1,0 | - | - | - | - |
| Chitosan | | | | | | | | | | |
| **Copherol® 12250** | - | - | 0,1 | 0,1 | - | - | - | - | - | - |
| Tocopherol Acetate | | | | | | | | | | |
| **Arlypon® F** | - | - | - | - | - | - | 3,0 | 3,0 | 1,0 | - |
| Laureth-2 | | | | | | | | | | |
| **Sodium Chloride** | - | - | - | - | - | - | - | 1,5 | - | 1,5 |

| **Zusammensetzung (INCI)** | **11** | **12** | **13** | **14** | **15** | **16** | **17** | **18** | **19** | **20** |
|---|---|---|---|---|---|---|---|---|---|---|
| **Texapon® NSO** | 20,0 | 20,0 | 12,4 | - | 25,0 | 11,0 | - | - | - | - |
| Sodium Laureth Sulfate | | | | | | | | | | |
| **Texapon® K 14 S** | - | - | - | - | - | - | - | - | 11,0 | 23,0 |
| Sodium Myreth Sulfate | | | | | | | | | | |
| **Texapon® SB 3** | - | - | - | - | - | 7,0 | - | - | - | - |
| Disodium Laureth Sulfosuccinate | | | | | | | | | | |
| **Plantacare® 818** | 5,0 | 5,0 | 4,0 | - | - | - | - | - | 6,0 | 4,0 |
| Coco Glucosides | | | | | | | | | | |
| **Plantacare® 2000** | - | - | - | - | 5,0 | 4,0 | - | - | - | - |
| Decyl Glucoside | | | | | | | | | | |
| **Plantacare® PS 10** | - | - | - | 40,0 | - | - | 16,0 | 17,0 | - | - |
| Sodium Laureth Sulfate (and) Coco Glucosides | | | | | | | | | | |
| **Dehyton® PK 45** | 20,0 | 20,0 | - | - | 8,0 | - | - | - | - | 7,0 |
| Cocamidopropyl Betaine | | | | | | | | | | |
| **Eumulgin® B1** | - | - | - | - | 1,0 | - | - | - | - | - |
| Ceteareth-12 | | | | | | | | | | |
| **Eumulgin® B2** | - | - | - | 1,0 | - | - | - | - | - | - |
| Ceteareth-20 | | | | | | | | | | |
| **Lameform® TGI** | - | - | - | 4,0 | - | - | - | - | - | - |
| Polyglyceryl-3 Isostearate | | | | | | | | | | |
| **Dehymuls® PGPH** | - | - | 1,0 | - | - | - | - | - | - | - |
| Polyglyceryl-2 Dipolyhydroxystearate | | | | | | | | | | |
| **Monomuls® 90-L 12** | - | - | - | - | - | - | - | - | 1,0 | 1,0 |
| Glyceryl Laurate | | | | | | | | | | |
| **Cetiol® HE** | - | 0,2 | - | - | - | - | - | - | - | - |
| PEG-7 Glyceryl Cocoate | | | | | | | | | | |
| **Eutanol® G** | - | - | - | 3,0 | - | - | - | - | - | - |
| Octyldodecanol | | | | | | | | | | |
| **Nutrilan® Keratin W** | - | - | - | - | - | - | - | - | 2,0 | 2,0 |
| Hydrolyzed Keratin | | | | | | | | | | |
| **Nutrilan® I** | 1,0 | - | - | - | - | 2,0 | - | 2,0 | - | - |
| Hydrolyzed Collagen | | | | | | | | | | |
| **Lamesoft® LMG** | - | - | - | - | - | - | - | - | 1,0 | - |
| Glyceryl Laurate (and) Potassium Cocoyl Hydrolyzed Collagen | | | | | | | | | | |
| **Lamesoft® 156** | - | - | - | - | - | - | - | - | - | 5,0 |
| Hydrogenated Tallow Gyceride (and) Potassium Cocoyl Hydrolyzed Collagen | | | | | | | | | | |
| **Gluadin® WK** | 1,0 | 1,5 | 4,0 | 1,0 | 3,0 | 1,0 | 2,0 | 2,0 | 2,0 | - |
| Sodium Cocoyl Hydrolyzed Wheat Protein | | | | | | | | | | |
| **Euperlan® PK 3000 AM** | 5,0 | 3,0 | 4,0 | - | - | - | - | 3,0 | 3,0 | - |
| Glycol Distearate (and) Laureth-4 (and) Cocamidopropyl Betaine | | | | | | | | | | |
| **Panthenol** | - | - | 1,0 | - | - | - | - | - | - | - |
| **Arlypon®** F | 2,6 | 1,6 | - | 1,0 | 1,5 | - | - | - | - | - |
| Laureth-2 | | | | | | | | | | |
| Rückfettungscompound gem. Bsp. 4 | 2,0 | 2,0 | 2,0 | 2,0 | 2,0 | 2,0 | 2,0 | 2,0 | 2,0 | 2,0 |
| **Cosmedia® Guar** | 0,1 | 0,2 | 0,3 | - | - | - | - | - | - | - |
| Guar Hydroxypropyl Trimonium Chloride | | | | | | | | | | |
| **Sodium Chloride** | - | - | - | - | - | 1,6 | 2,0 | 2,2 | - | 3,0 |
| **Glycerin** (86 Gew.-%ig) | - | 5,0 | - | - | - | - | - | 1,0 | 3,0 | - |

| | | | | | | | | | | |
|---|---|---|---|---|---|---|---|---|---|---|
| (1-4) Haarspülung, (5-6) Haarkur, (7-8) Duschbad, (9) Duschgel, (10) Waschlotion (11-14) Duschbad "Two-in-One", (15-20) Shampoo | | | | | | | | | | |

## Patentansprüche

1. Kosmetische Zubereitungen, bestehend aus
(a) 40 bis 99 Gew.-% Fettalkoholen der Formel **(I)**
**R**^{**1**}**OH** **(I)**
in der R¹ für einen aliphatischen, linearen oder verzweigten Kohlenwasserstoffrest mit 6 bis 22 Kohlenstoffatomen und 0 und/oder 1, 2 oder 3 Doppelbindungen steht und
(b) 1 bis 60 Gew.-% Wirkstoffen mit einer Wasserlöslichkeit bei 20 °C von weniger als 1 g/l, die ausgewählt sind aus der Gruppe, die gebildet wird von Fettsäuren, Ölkörpern, Fetten und Wachsen, Partialglyceriden sowie biogenen aktiven Systemen,
mit der Maßgabe, dass sich die Mengenangaben zu 100 Gew.-% ergänzen.

2. Zubereitungen gemäß Anspruch 1, **dadurch gekennzeichnet, dass** sie als Komponente (b1) Fettsäuren der Formel **(II)** enthalten,
**R**^{**2**}**CO-OH** **(II)**
in der R²CO für einen aliphatischen, linearen oder verzweigten Acylrest mit 6 bis 22 Kohlenstoffatomen und 0 und/oder 1, 2 oder 3 Doppelbindungen steht.

3. Zubereitungen: nach mindestens einem der Ansprüche 1 bis 2, **dadurch gekennzeichnet, dass** sie als Komponente (b2) Ölkörper enthalten, die ausgewählt sind aus der Gruppe, die gebildet wird von Guerbetalkohoien auf Basis von Fettalkoholen mit 6 bis 18 Köhlehstoffatomen, Estern von linearen C₆-C₂₂-Fettsäuren mit linearen oder verzweigten C₆-C₂₂-Fettalkoholen bzw. Estern von verzweigten C₆-C₁₃-Carbonsäuren mit linearen oder verzweigten C₆-C₂₂-Fettalkoholen, Estern von linearen C₆-C₂₂-Fettsäuren mit verzweigten Alkoholen, Estern von C₁₈-C₃₈-Alkylhydroxycarborisäuren mit linearen oder verzweigten C₆-C₂₂-Fettalkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈₋Fettsäuren, Sheabutter, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primären Alkoholen, substituierten Cyclohexanen, linearen und verzweigten C₆-C₂₂₋Fettalkoholcarbonaten, Guerbetcarbonaten auf Basis von Fettalkoholen mit 6 bis 18 C Atomen, Estern der Benzoesäure mit linearen und/oder verzweigten C₆-C₂₂-Alkoholen, linearen oder verzweigten, symmetrischen oder unsymmetrischen Dialkylethern mit 6 bis 22 Kohlenstoffatomen pro Alkylgruppe, Ringöffnungsprodukten von epoxidierten Fettsäureestern mit Polyolen und aliphatischen bzw, naphthenischen Kohlenwasserstoffen.

4. Zubereitungen nach mindestens einem der Ansprüche 1 bis 3, **dadurch gekennzeichnet, dass** sie als Komponente (b3) Fette bzw. Wachse enthalten, die ausgewählt sind aus der Gruppe, die gebildet wird von Glycerinestern höherer Fettsäuren mit 12 bis 22 Kohlenstoffatomen, Lecithinen, Phospholipiden, Sphingolipiden, Candellilawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachsen, Mikrowachsen, Montanesterwachsen, Sasolwachsen, hydrierten Jojobawachsen, Polyalkylenwachsen und Polyethylenglycolwachsen.

5. Zubereitungen nach mindestens einem der Ansprüche 1 bis 4, **dadurch gekennzeichnet, dass** sie als Komponente (b4) Siliconverbindungen enthalten, die ausgewählt sind aus der Gruppe, die gebildet wird von Dimethylpolysiloxanen, Methylphenylpolysiloxanen, cyclischen Siliconen sowie amino-, fettsäure-, alkohol-, polyether-, epoxy-, fluor-, glykosid- und/oder alkylmodifizierte Siliconverbindungen.

6. Zubereitungen nach mindestens einem der Ansprüche 1 bis 5, **dadurch gekennzeichnet, dass** sie als Komponente (b5) Partialgtycerlde der Formel **(III)** enthalten, 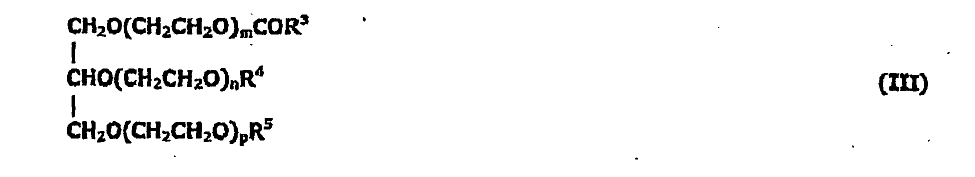 in der R³CO für einen linearen oder verzweigten, gesättigten und/oder ungesättigten Acylrest mit 6 bis 22 Kohlenstoffatomen, R⁴ und R⁵ unabhängig voneinander für R³CO oder OH und die Summe (m+n+p) für 0 oder Zahlen von 1 bis 100 steht, mit der Maßgabe, dass mindestens einer der beiden Reste R⁴ und R⁵ OH bedeutet.

7. Zubereitungen nach mindestens einem der Ansprüche 1 bis 6, **dadurch gekennzeichnet, dass** sie als Komponente (b6) biogene aktive Systeme enthalten, die ausgewählt sind aus der Gruppe, die gebildet wird von Tocopherolen, Tocopherolestern, Sterolen, Sterolestern und Ceramiden.

## Claims

1. Cosmetic preparations consisting of
(a) 40 to 99% by weight of fatty alcohols corresponding to formula(I):
**R**^{**1**}**OH** **(I)**
where R¹ is an aliphatic, linear or branched hydrocarbon radical containing 6 to 22 carbon atoms and 0 and/or 1, 2 or 3 double bonds,
and
(b) 1 to 60% by weight of active substances with a solubility in water at 20°C of less than 1 g/l selected from the group consisting of fatty acids, oil components, fats and waxes, partial glycerides and biogenic active systems,
with the proviso that the quantities shown add up to 100% by weight.

2. Preparations as claimed in claim 1, **characterized in that** they contain fatty acids corresponding to formula (II):
**R**^{**2**}**CO-OH** **(II)**
in which R²CO is an aliphatic, linear or branched acyl group containing 6 to 22 carbon atoms and 0 and/or 1, 2 or 3 double bonds, as component (b1).

3. Preparations as claimed in at least one of claims 1 and 2, **characterized in that** they contain as component (b2) oil components selected from the group consisting of Guerbet alcohols based on fatty alcohols containing 6 to 18 carbon atoms, esters of linear C₆₋₂₂ fatty acids with linear or branched C₆₋₂₂ fatty alcohols or esters of branched C₆₋₁₃ carboxylic acids with linear or branched C₆₋₂₂ fatty alcohols, esters of linear C₆₋₂₂ fatty acids with branched alcohols, esters of C₁₈₋₃₈ alkyl hydroxycarboxylic acids with linear or branched C₆₋₂₂ fatty alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, shea butter, esters of C₆₋₂₂ fatty alcohols and/or Guerbet alcohols with aromatic carboxylic acids, esters of C₂₋₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear and branched C₆₋₂₂ fatty alcohol carbonates, Guerbet carbonates based on C₆₋₁₈ fatty alcohols, esters of benzoic acid with linear and/or branched C₆₋₂₂ alcohols, linear or branched, symmetrical or nonsymmetrical dialkyl ethers containing 6 to 22 carbon atoms per alkyl group, ring opening products of epoxidized fatty acid esters with polyols and aliphatic or naphthenic hydrocarbons.

4. Preparations as claimed in at least one of claims 1 to 3, **characterized in that** they contain as component (b3) fats or waxes selected from the group consisting of glycerol esters of higher fatty acids containing 12 to 22 carbon atoms, lecithins, phospholipids, sphingolipids, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes, montan ester waxes, sasol waxes, hydrogenated jojoba waxes, polyalkylene waxes and polyethylene glycol waxes.

5. Preparations as claimed in at least one of claims 1 to 4, **characterized in that** they contain as component (b4) silicone compounds selected from the group consisting of dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds as component (b4).

6. Preparations as claimed in at least one of claims 1 to 5, **characterized in that** they contain partial glycerides corresponding to formula **(III):** 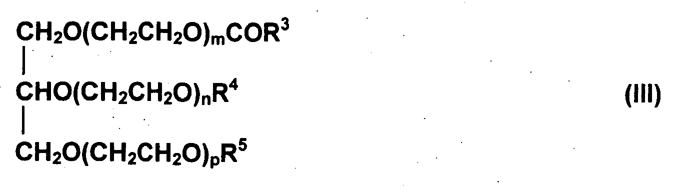 where R³CO is a linear or branched, saturated and/or unsaturated acyl group containing 6 to 22 carbon atoms, R⁴ and R⁵ independently of one another have the same meaning as R³CO or represent OH and the sum (m+n+p) is 0 or a number of 1 to 100, with the proviso that at least one of the two substituents R⁴ and R⁵ is OH,
as component (b5).

7. Preparations as claimed in at least one of claims 1 to 6, **characterized in that** they contain biogenic active systems selected from the group consisting of tocopherols, tocopherol esters, sterols, sterol esters and ceramides as component (b6).

## Revendications

1. Préparations cosmétiques composées de
(a) 40 à 99 % en poids d'alcools gras de formule (I)
R¹OH (I)
dans laquelle R¹ représente un radical hydrocarboné aliphatique, linéaire ou ramifié ayant de 6 à 22 atomes de carbone et 0 et/ou 1, 2 ou 3 doubles liaisons, et
(b) 1 à 60 % en poids de principes actifs ayant une solubilité dans l'eau à 20°C de moins de 1 g/l choisis dans le groupe constitué par les acides gras, les corps huileux, les graisses et les cires, les glycérides partiels ainsi que des systèmes actifs biogènes,
étant precisé que les quantités données se complètent à 100 % en poids.

2. Préparations selon la revendication 1,
**caractérisées en ce qu'**
elles contiennent comme composant (b1) des acides gras de formule (II),
R²CO-OH (II)
dans laquelle R²CO représente un radical acyle aliphatique, linéaire ou ramifié ayant de 6 à 22 atomes de carbone et 0 et/ou 1, 2 ou 3 doubles liaisons.

3. Préparations selon l'une au moins des revendications 1 et 2,
**caractérisées en ce qu'**
elles contiennent comme composant (b2) des corps huileux choisis dans le groupe constitué par les alcools de Guerbet à base d'alcools gras ayant de 6 à 18 atomes de carbone, les esters d'acides gras en C₆-C₂₂ linéaires et d'alcools gras en C₆-C₂₂ linéaires, ou ramifiés ou les esters d'acides carboxyliques en C₆-C₁₃ ramifiés et d'alcools gras en C₆-C₂₂ linéaires ou ramifiés, les esters d'acides gras en C₆-C₂₂ linéaires et d'alcools ramifiés, les esters d'acides alkylhydroxycarboxyliques en C₁₈₋C₃₈ et d'alcools gras en C₆-C₂₂ linéaires ou ramifiés, les esters d'acides gras linéaires et/ou ramifiés et d'alcools de Guerbet et/ou d'alcools polyvalents, les triglycérides à base d'acides gras en C₆-C₁₀, les mélanges de monoglycérides/diglycérides/triglycérides liquides à base d'acides gras en C₆-C₁₈, le beurre de karité, les esters d'alcools gras en C₆-C₂₂ et/ou d'alcools de Guerbet et d'acides carboxyliques aromatiques, les esters d'acides dicarboxyliques en C₂-C₁₂ et d'alcools linéaires ou ramifiés ayant de 1 à 22 atomes de carbone ou de polyols ayant de 2 à 10 atomes de carbone et de 2 à 6 groupes hydroxyle, les huiles végétales, les alcools primaires ramifiés, les cyclohexanes substitués, les carbonates d'alcools gras en C₆-C₂₂ linéaires ou ramifiés, les carbonates de Guerbet à base d'alcools gras ayant de 6 à 18 atomes de carbone, les esters de l'acide benzoïque avec des alcools en C₆-C₂₂ linéaires et/ou ramifiés, les dialkyléthers linéaires ou ramifiés, symétriques ou asymétriques ayant de 6 à 22 atomes de carbone par groupe alkyle, des produits d'ouverture de cycle d'esters d'acide gras époxydés avec des polyols et les hydrocarbures aliphatiques ou naphténiques.

4. Préparations selon l'une au moins des revendications 1 à 3,
**caractérisées en ce qu'**
elles contiennent comme composant (b3) des graisses ou cires choisies dans le groupe constitué par les esters de glycérine et d'acides gras supérieurs ayant de 12 à 22 atomes de carbone, les lécithines, les phospholipides, les sphingolipides, la cire de Candelilla, la cire de Carnauba, la cire du Japon, la cire d'Esparto, la cire de fibres de liège, la cire de Guaruma, la cire d'huile de germe de riz, la cire de canne à sucre, la cire d'Ouricury, la cire de Montan, la cire d'abeilles, la cire de laque, le spermaceti, la lanoline (cire de laine), la graisse de croupion, la cérésine, l'ozokérite (cire de terre), la vaseline, les cires de paraffine, les microcires, les cires d'ester de Montan, les cires Sasol, les cires de jojoba hydrogénées, les cires de polyalkylène et les cires de polyéthylèneglycol.

5. Préparations selon l'une au moins des revendications 1 à 4,
**caractérisées en ce qu'**
elles contiennent comme composant (b4) des composés de silicone choisis dans le groupe constitué par les diméthylpolysiloxanes, les méthylphénylpolysiloxanes, les silicones cycliques ainsi que les composés de silicone modifiés par des fonctions amine, acide gras, alcool, polyéther, époxyde, fluor, glycoside et/ou alkyle.

6. Préparations selon l'une au moins des revendications 1 à 5,
**caractérisées en ce qu'**
elles contiennent comme composant (b5) des glycérides partiels de formule (III) 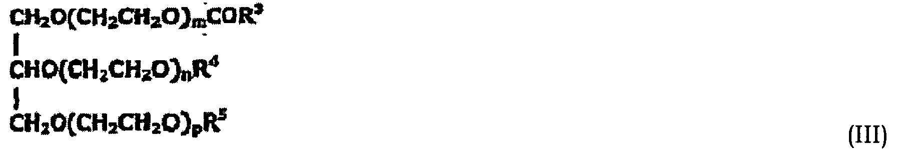 dans laquelle R³CO représente un radical acyle linéaire ou ramifié, saturé et/ou insaturé ayant de 6 à 22 atomes de carbone, R⁴ et R⁵ représentent indépendamment l'un de l'autre un groupe R³CO ou OH et la somme (m+n+p) est égale à 0 ou à des nombres allant de 1 à 100, etant précisé qu'au moins l'un des deux radicaux R⁴ et R⁵ représente OH.

7. Préparations selon l'une au moins des revendications 1 à 6,
**caractérisées en ce qu'**
elles contiennent comme composant (b6) des systèmes actifs biogènes choisis dans le groupe constitué par les tocophérols, les esters de tocophérol, les stérols, les esters de stérol et les céramides.
